# EUROPEAN PATENT SPECIFICATION

(11) **EP 2 417 152 B1**
(45) Date of publication and mention of the grant of the patent: **02.10.2013**
(21) Application number: 10715185.4
(22) Date of filing: 07.04.2010
(51) Int. Cl.: C07K 7/08, C07K 14/245

(54) **PROCESS FOR ISOLATING LINACLOTIDE**
VERFAHREN ZUR ISOLIERUNG VON LINACLOTID
PROCÉDÉ POUR L'ISOLEMENT DE LA LINACLOTIDE

(30) Priority: 10.04.2009 US 168259 P
(43) Date of publication of application: 15.02.2012
(73) Proprietor: Corden Pharma Colorado, Inc., Boulder, CO 80301 (US)
(72) Inventor: FLEMING, Michael Paul, Longmont Colorado 80051 (US); PETERSEN, Raymond Dennis, Thornton Colorado 80229 (US)
(74) Representative: Vossius & Partner
(86) International application number: PCT/EP2010/054577
(87) International publication number: WO 2010/115916

(56) References cited:
- WO-A1-2005/087797
- WO-A2-2004/069165

## Description

This invention relates to methods of isolating a peptide for treating various disorders, including gastrointestinal disorders, obesity, congestive heart failure and benign prostatic hyperplasia.

Irritable bowel syndrome (IBS) is a common chronic disorder of the intestine that affects 20 to 60 million individuals in the US alone. IBS is the most common disorder diagnosed by gastroenterologists (28% of patients examined) and accounts for 12% of visits to primary care physicians (Camilleri 2001, Gastroenterology 120:652-668). In the US, the economic impact of IBS is estimated at $25 billion annually, through direct costs of health care use and indirect costs of absenteeism from work (Talley 1995, Gastroenterology 109:1736-1741). Patients with IBS have three times more absenteeism from work and report a reduced quality of life. Sufferers may be unable or unwilling to attend social events, maintain employment, or travel even short distances (Drossman 1993, Dig Dis Sci 38:1569-1580). There is a tremendous unmet medical need in this population since few prescription options exist to treat IBS.

Linaclotide is a first-in-class 14mer peptide and a guanylate cyclase type C receptor (GCC) agonist that stimulates the production of cyclic guanosine monophosphate (cGMP). In vitro studies suggest that linaclotide increased cGMP in human intestinal cells at varying concentrations, and in vivo studies in mice and rats showed that oral linaclotide had promising effects on increasing small bowel motility, fluid secretion and decreasing visceral pain.

Linaclotide is a 14-amino-acid cyclic peptide with three disulfide bonds, the sequence consisting of cyclized (SEQ ID NO. 1)

Cys-Cys-Glu-Tyr-Cys-Cys-Asn-Pro-Ala-Cys-Thr-Gly-Cys-Tyr

with disulfide bridges between the cysteine residues at positions 1 and 6, 2 and 10, and 5 and 13 as shown below:

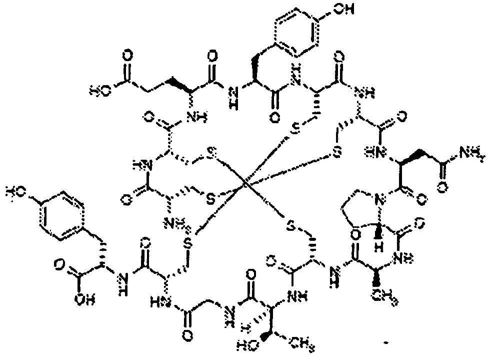

It shares common pharmacological activity in the GI tract with the endogenous hormones GN and UGN (GASTROENTEROLOGY 2005 128 4 Suppl 2 A464). The drug acts as a GCC superagonist, thereby elevating intracellular cGMP.

The cyclopeptide is typically synthesized linearly using an automated solid-phase peptide synthesizer prior to forming the disulfide bonds (US7,371,727; WO-2004069165; Current Opinion in Molecular Therapeutics 2007 Vol. 9 No. 4). However, the typical linear build combined with lyophilization as the method of isolation results in a 10-20% yield (US7,371,727). Lyophilization does not provide bulk material with properties optimal for manufacture. The bulk density of lyophilized material is usually low, the material has poor handling characteristics (fine particulate subject to static charging) and potential reduced stability due to high surface area. Further, the potency of current pharmaceutical compounds engenders industrial hygiene concerns associated with the open handling of lyophilized materials. Thus, improved methods of isolation of linaclotide, cyclized (SEQ ID NO 1), are necessary.

In a first object, the present invention provides a process for precipitating a peptide comprising the step of:
a) providing an aqueous isopropanol or ethanol solution of a cyclized peptide including the amino acid sequence of(SEQ ID NO. 1)

   Cys-Cys-Glu-Tyr-Cys-Cys-Asn-Pro-Ala-Cys-Thr-Gly-Cys-Tyr

In a preferred embodiment, the alcohol solution is an isopropanol solution and the process further comprising the step of:
b) feed-stripping the solution resulting from step a) with isopropanol in order to reduce the percentage of water in the solution.

In a further preferred embodiment, the alcohol solution is an ethanol solution and the process further comprising the step of:
b) feed-stripping the solution resulting from step a) with ethanol in order to reduce the percentage of water in the solution.

In a further preferred embodiment, the aqueous isopropanol solution is an approximately 45% to approximately 70% aqueous isopropanol solution.

In a further preferred embodiment, the aqueous isopropanol solution is an approximately 45% to approximately 65% aqueous isopropanol solution.

In a further preferred embodiment, the aqueous isopropanol solution is an approximately 45% to approximately 60% aqueous isopropanol solution.

In a further preferred embodiment, the aqueous isopropanol solution is an approximately 50% to approximately 60% aqueous isopropanol solution.

In a further preferred embodiment, the aqueous isopropanol solution is an approximately 55% aqueous isopropanol solution.

In a further preferred embodiment, the aqueous ethanol solution is an approximately 55% to approximately 75% aqueous ethanol solution.

In a further preferred embodiment, the aqueous ethanol solution is an approximately 60% to approximately 75% aqueous ethanol solution.

In a further preferred embodiment, the aqueous ethanol solution is an approximately 65% to approximately 75% aqueous ethanol solution.

In a further preferred embodiment, the feed-stripping with isopropanol or ethanol in step b) reduces the percentage of water in the solution until the percentage of isopropanol or ethanol is greater than 96%, preferably 97%, more preferably 98%.

In a further preferred embodiment, the process of the present invention further comprises the step of:
c) feed-stripping the solution resulting from step b) with heptane.

In a further preferred embodiment, the feed-stripping with heptane in step c) reduces the percentage of isopropanol or ethanol in the solution until the percentage of heptane is greater than 98%, preferably 98.5 %, more preferably 99%, even more preferably 99.5%, most preferably 99.9%.

In an alternative embodiment, the process of the present invention further comprises the step of:
b) diluting with water the solution resulting from step a).

In a further preferred embodiment, the solution is diluted to about 3/1 v/v water/isopropanol or to about 3/1 v/v water/ethanol to precipitate SEQ ID No. 1.

In a second object, the present invention provides a process for isolating a peptide comprising the steps of:
a) providing an aqueous isopropanol or ethanol solution of a cyclized peptide including the amino acid sequence of (SEQ ID NO. 1)

   Cys-Cys-Glu-Tyr-Cys-Cys-Asn-Pro-Ala-Cys-Thr-Gly-Cys-Tyr
b) spray drying the solution resulting from step a).

In a further preferred embodiment, the aqueous isopropanol solution is an approximately 45% to approximately 70% aqueous isopropanol solution.

In a further preferred embodiment, the aqueous isopropanol solution is an approximately 45% to approximately 65% aqueous isopropanol solution.

In a further preferred embodiment, the aqueous isopropanol solution is an approximately 45% to approximately 60% aqueous isopropanol solution.

In a further preferred embodiment, the aqueous isopropanol solution is an approximately 50% to approximately 60% aqueous isopropanol solution.

In a further preferred embodiment, the aqueous isopropanol solution is an approximately 55% aqueous isopropanol solution.

In a further preferred embodiment, the aqueous ethanol solution is an approximately 55% to approximately 75% aqueous ethanol solution.

In a further preferred embodiment, the aqueous ethanol solution is an approximately 60% to approximately 75% aqueous ethanol solution.

In a further preferred embodiment, the aqueous ethanol solution is an approximately 65% to approximately 75% aqueous ethanol solution.

### Definitions

The phrase "a" or "an" entity as used herein refers to one or more of that entity; for example, a compound refers to one or more compounds or at least one compound. As such, the terms "a" (or "an"), "one or more", and "at least one" can be used interchangeably herein.

As used in this specification, whether in a transitional phrase or in the body of the claim, the terms "comprise(s)" and "comprising" are to be interpreted as having an open-ended meaning. That is, the terms are to be interpreted synonymously with the phrases "having at least" or "including at least". When used in the context of a process, the term "comprising" means that the process includes at least the recited steps, but may include additional steps. When used in the context of a compound or composition, the term "comprising" means that the compound or composition includes at least the recited features or components, but may also include additional features or components. As used herein, unless specifically indicated otherwise, the word "or" is used in the "inclusive" sense of "and/or" and not the "exclusive" sense of "either/or".

The term "about" is used herein to mean approximately, in the region of, roughly, or around. When the term "about" is used in conjunction with a numerical range, it modifies that range by extending the boundaries above and below the numerical values set forth.

As used herein, the terms "cyclized peptide of SEQ ID No 1", "cyclized peptide including the amino acid sequence of (SEQ ID NO. 1)", "linaclotide", and "a 14-amino-acid cyclic peptide with three disulfide bonds, the sequence consisting of cyclized (SEQ ID NO. 1)" means the peptide sequence of (SEQ ID NO. 1)

Cys-Cys-Glu-Tyr-Cys-Cys-Asn-Pro-Ala-Cys-Thr-Gly-Cys-Tyr

wherein the peptide of (SEQ ID NO. 1) is folded in the poly-cyclic tri-disulfide form due to disulfide bridges between the cysteine residues at positions 1 and 6, 2 and 10, and 5 and 13.

Technical and scientific terms used herein have the meaning commonly understood by one of skill in the art to which the present invention pertains, unless otherwise defined. Reference is made herein to various methodologies and materials known to those of skill in the art. Standard reference works setting forth the general principles of pharmacology include Goodman and Gil-man's The Pharmacological Basis of Therapeutics, 10th Ed., McGraw Hill Companies Inc., New York (2001). Any suitable materials and/or methods known to those of skill can be utilized in carrying out the present invention. However, preferred materials and methods are described. Materials, reagents and the like to which reference are made in the following description and examples are obtainable from commercial sources, unless otherwise noted.

As used herein, the term "including the amino acid sequence" preferably means "having the amino acid sequence".

As used herein, the phrase "approximately 45% to approximately 70% aqueous isopropanol solution" means a solution comprising between approximately 45% v/v isopropanol and approximately 55% v/v water to approximately 70% v/v isopropanol and approximately 30% v/v water. As used herein, in this context, the term "approximately" means plus or minus 5% v/v.

As used herein, the phrase "approximately 45% to approximately 60% aqueous isopropanol solution" means a solution comprising between approximately 45% v/v isopropanol and approximately 55% v/v water to approximately 60% v/v isopropanol and approximately 40% v/v water. As used herein, in this context, the term "approximately" means plus or minus 5% v/v.

As used herein, the phrase "approximately 50% to approximately 60% aqueous isopropanol solution" means a solution comprising between approximately 50% v/v isopropanol and approximately 50% v/v water to approximately 60% v/v isopropanol and approximately 40% v/v water. As used herein, in this context, the term "approximately" means plus or minus 5% v/v.

As used herein, the phrase "approximately 55% aqueous isopropanol solution" means a solution comprising approximately 55% v/v isopropanol and approximately 45% v/v water. As used herein, in this context, the term "approximately" means plus or minus 5% v/v.

As used herein, the phrase "approximately 55% to approximately 75% aqueous ethanol solution" means a solution comprising between approximately 55% v/v ethanol and approximately 45% v/v water to approximately 75% v/v ethanol and approximately 25% v/v water. As used herein, in this context, the term "approximately" means plus or minus 5% v/v.

As used herein, the phrase "approximately 60% to approximately 75% aqueous ethanol solution" means a solution comprising between approximately 60% v/v ethanol and approximately 40% v/v water to approximately 75% v/v ethanol and approximately 25% v/v water. As used herein, in this context, the term "approximately" means plus or minus 5% v/v.

As used herein, the phrase "approximately 65% to approximately 75% aqueous ethanol solution" means a solution comprising between approximately 65% v/v ethanol and approximately 35% v/v water to approximately 75% v/v ethanol and approximately 25% v/v water. As used herein, in this context, the term "approximately" means plus or minus 5% v/v.

As used herein, the phrase "approximately 65% to approximately 70% aqueous ethanol solution" means a solution comprising between approximately 65% v/v ethanol and approximately 35% v/v water to approximately 70% v/v ethanol and approximately 30% v/v water. As used herein, in this context, the term "approximately" means plus or minus 5% v/v.

As used herein, the phrase "approximately 67% aqueous ethanol solution" means a solution comprising approximately 67% v/v ethanol and approximately 33% v/v water. As used herein, in this context, the term "approximately" means plus or minus 5% v/v.

As used herein, the phrase "feed stripping the solution" means that a second solvent is gradually added under vacuum to a first solution and the second solvent gradually replaces the first solution with the second solvent while maintaining approximately constant volume. For example, feed stripping a 50% v/v aqueous isopropanol solution with isopropanol would gradually result in an isopropanol solution, wherein the remaining water is less than approximately 2%. For example, feed stripping an isopropanol solution with heptane would gradually result in a heptane solution, wherein the remaining isopropanol is less than approximately 0.1%. For example, feed stripping a 50% v/v aqueous ethanol solution with ethanol would gradually result in an ethanol solution, wherein the remaining water is less than approximately 2%. For example, feed stripping an ethanol solution with heptane would gradually result in a heptane solution, wherein the remaining ethanol is less than approximately 0.1%.

As used herein, the term "diluting with water" means adding water to a solution to increase the percentage by volume of water such that the resulting solution is about 3/1 v/v water to alcohol. For example, diluting a 50% v/v aqueous isopropanol solution with water would yield a solution of approximately 3/1 v/v water/isopropanol. For example, diluting a 50% v/v aqueous ethanol solution with water would yield a solution of approximately 3/1 v/v water/ethanol.

As used herein, "large scale" with respect to peptide synthesis generally includes the synthesis of peptides in the range of at least 500 g, more preferably at least 2 kg per batch. Largescale synthesis is typically performed in large reaction vessels, such as steel reaction vessels, that can accommodate quantities of reagents such as resins, solvents, amino acids, chemicals for coupling, and deprotection reactions, that are sized to allow for production of peptides in the kilogram to metric ton range.

Commonly used abbreviations include: acetyl (Ac), azo-bis-isobutyrylnitrile (AIBN), atmospheres (Atm), 9-borabicyclo[3.3.1]nonane (9-BBN or BBN), tert-butoxycarbonyl (Boc), di-tert-butyl pyrocarbonate or boc anhydride (BOC2O), benzyl (Bn), butyl (Bu), Chemical Abstracts Service Registration Number (CASRN), benzyloxycarbonyl (CBZ or Z), carbonyl diimidazole (CDI), 1,4-diazabicyclo[2.2.2]octane (DABCO), diethylaminosulfur trifluoride (DAST), dibenzylideneacetone (dba), 1,5-diazabicyclo[4.3.0]non-5-ene (DBN), 1,8-diazabicyclo[5.4.0]undec-7-ene (DBU), N,N'-dicyclohexylcarbodiimide (DCC), 1,2-dichloroethane (DCE), dichloromethane (DCM), diethyl azodicarboxylate (DEAD), di-iso-propylazodicarboxylate (DIAD), di-iso-butylaluminumhydride (DIBAL or DIBAL-H), di-isopropylethylamine (DIPEA), N,N-dimethyl acetamide (DMA), 4-N,N-dimethylaminopyridine (DMAP), N,N-dimethylformamide (DMF), dimethyl sulfoxide (DMSO), 1,1'-bis-(diphenylphosphino)ethane (dppe), 1,1'-bis-(diphenylphosphino)ferrocene (dppf), 1-(3-dimethylaminopropyl)-3-ethylcarbodiimide hydrochloride (EDCI), ethyl (Et), ethyl acetate (EtOAc), ethanol (EtOH), 2-ethoxy-2H-quinoline-1-carboxylic acid ethyl ester (EEDQ), diethyl ether (Et2O), O-(7-azabenzotriazole-1-yl)-N, N,N'N'-tetramethyluroniurn hexafluorophosphate (HATU), acetic acid (HOAc), 1-N-hydroxybenzotriazole (HOBt), high pressure liquid chromatography (HPLC), iso-propanol (IPA), lithium hexamethyl disilazane (LiHMDS), methanol (MeOH), melting point (mp), MeSO2- (mesyl or Ms), , methyl (Me), acetonitrile (MeCN), m-chloroperbenzoic acid (MCPBA), mass spectrum (ms), methyl t-butyl ether (MTBE), N-bromosuccinimide (NBS), N-carboxyanhydride (NCA), N-chlorosuccinimide (NCS), N-methylmorpholine (NMM), N-methylpyrrolidone (NMP), pyridinium chlorochromate (PCC), pyridinium dichromate (PDC), phenyl (Ph), propyl (Pr), iso-propyl (i-Pr), pounds per square inch (psi), pyridine (pyr), room temperature (rt or RT), tert-butyldimethylsilyl or t-BuMe2Si (TBDMS), triethylamine (TEA or Et3N), 2,2,6,6-tetramethylpiperidine 1-oxyl (TEMPO), triflate or CF3SO2- (Tf), trifluoroacetic acid (TFA), 1,1'-bis-2,2,6,6-tetramethylheptane-2,6-dione (TMHD), O-benzotriazol-1-yl-N,N,N',N'-tetramethyluronium tetrafluoroborate (TBTU), thin layer chromatography (TLC), tetrahydrofuran (THF), trimethylsilyl or Me3Si (TMS), p-toluenesulfonic acid monohydrate (TsOH or pTsOH), 4-Me-C6H4SO2- or tosyl (Ts), N-urethane-N-carboxyanhydride (UNCA),. Conventional nomenclature including the prefixes normal (n), iso (i-), secondary (sec-), tertiary (tert-) and neo have their customary meaning when used with an alkyl moiety. (J. Rigaudy and D. P. Klesney, Nomenclature in Organic Chemistry, IUPAC 1979 Pcrgamon Press, Oxford.).

Linaclotide may be synthesized linearly using standard automated solid phase synthesis equipment (US7,371,727, WO2004069165, Current Opinion in Molecular Therapeutics 2007 9(4):403-410).

After the peptide of SEQ ID No. 1 is formed, the product can be subject to, purification, lyophilization, further processing (e.g., reaction with another peptide to form a fusion protein); combinations of these, and/or the like, as desired.

For example, in the current process, the peptide is oxidized to form the tri-disulfide form before purification.

Specifically, the linear peptide is formed on resin and the crude peptide then cleaved from the resin in the linear thiol form. The linear form is then folded (and oxidized) to form the poly-cyclic tri-disulfide form. This tri-disulfide form is then purified by HPLC and the purified pools are concentrated (again by chromatography) to a smaller volume pool in water/isopropanol. This solution is then taken into the precipitation for isolation.

After purification, the peptide of SEQ ID NO. 1 may be lyophilized or precipitated out of solution. Because lyophilization is not amenable to large scale manufacture of peptides as required for commercial production, the present application disclosed useful and novel methods for precipitating purified linaclotide, as opposed to lyophilization of the purified peptide.

In one preferred embodiment, cyclized SEQ ID NO. 1 is dissolved in an aqueous isopropanol solution. The solution may contain residual amounts of acetic acid and trifluoroacetic acid from HPLC chromatography. In the following, all percentages and ratios are by volume unless otherwise expressly stated.

In one preferred embodiment, SEQ ID NO. 1 is dissolved in an aqueous isopropanol solution wherein the percent isopropanol component of the solution is between approximately 45% and approximately 70% v/v, and the remainder of the solution is water.

In one preferred embodiment, SEQ ID NO. 1 is dissolved in an aqueous isopropanol solution wherein the percent isopropanol component of the solution is between approximately 45% and approximately 60% v/v, and the remainder of the solution is water.

In one preferred embodiment, SEQ ID NO. 1 is dissolved in an aqueous isopropanol solution wherein the percent isopropanol component of the solution is between approximately 50% and approximately 60% v/v, and the remainder of the solution is water.

In one preferred embodiment, SEQ ID NO. 1 is dissolved in an aqueous isopropanol solution wherein the percent isopropanol component of the solution is between approximately 50% and approximately 55% v/v, and the remainder of the solution is water.

In one preferred embodiment, SEQ ID NO. 1 is dissolved in an aqueous isopropanol solution wherein the percent isopropanol component of the solution is between approximately 50% and approximately 52.5% v/v, and the remainder of the solution is water.

In one preferred embodiment, SEQ ID NO. 1 is dissolved in an aqueous isopropanol solution wherein the percent isopropanol component of the solution is between approximately 52.5% and approximately 55% v/v, and the remainder of the solution is water.

In a preferred embodiment, the percent isopropanol component of the solution is approximately 55%v/v, and the remainder of the solution is water.

In a preferred embodiment, the isopropanol solution containing SEQ ID No. 1 is then diluted to about 3/1 v/v water/isopropanol to precipitate SEQ ID No. 1.

In an alternative embodiment, the aqueous isopropanol solution containing SEQ ID NO. 1 is, instead of diluting with water, feed-stripped with isopropanol in order to reduce the percentage of water in the solution until the percentage of isopropanol is greater than 95%.

In another alternative embodiment, the aqueous isopropanol solution containing SEQ ID NO. 1 is then feed-stripped with isopropanol in order to reduce the percentage of water in the solution until the percentage of isopropanol is greater than 96%.

In another alternative embodiment, the aqueous isopropanol solution containing SEQ ID NO. 1 is then feed-stripped with isopropanol in order to reduce the percentage of water in the solution until the percentage of isopropanol is greater than 97%.

In another alternative embodiment, the aqueous isopropanol solution containing SEQ ID NO. 1 is then feed-stripped with isopropanol in order to reduce the percentage of water in the solution until the percentage of isopropanol is greater than 98%.

In another alternative embodiment, the isopropanol solution containing SEQ ID NO. 1 is subsequently feed-stripped with heptane in order to reduce the percentage of isopropanol in the solution until the percentage of heptane is greater than 98%.

In another alternative embodiment, the isopropanol solution containing SEQ ID NO. 1 is subsequently feed-stripped with heptane in order to reduce the percentage of isopropanol in the solution until the percentage of heptane is greater than 98.5%.

In another alternative embodiment, the isopropanol solution containing SEQ ID NO. 1 is subsequently feed-stripped with heptane in order to reduce the percentage of isopropanol in the solution until the percentage of heptane is greater than 99%.

In another alternative embodiment, the isopropanol solution containing SEQ ID NO. I is subsequently feed-stripped with heptane in order to reduce the percentage of isopropanol in the solution until the percentage of heptane is greater than 99.5%.

In another alternative embodiment, the isopropanol solution containing SEQ ID NO. 1 is subsequently feed-stripped with heptane in order to reduce the percentage of isopropanol in the solution until the percentage of heptane is greater than 99.9%.

In another preferred alternative embodiment, the aqueous isopropanol solution containing SEQ ID NO 1, is instead of diluting with water or feedstripping with isopropanol and then heptane, spray-dried.

In one preferred method, SEQ ID NO. 1 is dissolved in an aqueous ethanol solution. The solution may contain residual amounts of acetic acid and trifluoroacetic acid from HPLC chromatography.

In one preferred method, SEQ ID NO. 1 is dissolved in an aqueous ethanol solution wherein the percent ethanol component of the solution is between approximately 55% and approximately 75%v/v, and the remainder of the solution is water.

In one preferred method, SEQ ID NO. 1 is dissolved in an aqueous ethanol solution wherein the percent ethanol component of the solution is between approximately 60% and approximately 75%v/v, and the remainder of the solution is water.

In one preferred method, SEQ ID NO. 1 is dissolved in an aqueous ethanol solution wherein the percent ethanol component of the solution is between approximately 65% and approximately 75%v/v, and the remainder of the solution is water.

In one preferred method, SEQ ID NO. 1 is dissolved in an aqueous ethanol solution wherein the percent ethanol component of the solution is between approximately 65% and approximately 70%v/v, and the remainder of the solution is water.

In one preferred method, SEQ ID NO. 1 is dissolved in an aqueous ethanol solution wherein the percent ethanol component of the solution is between approximately 65% and approximately 67%v/v, and the remainder of the solution is water.

In one preferred method, SEQ ID NO. 1 is dissolved in an aqueous ethanol solution wherein the percent ethanol component of the solution is between approximately 67% and approximately 70% v/v, and the remainder of the solution is water.

In a preferred embodiment, the percent ethanol component of the solution is approximately 67%v/v, and the remainder of the solution is water.

In a preferred embodiment, the ethanol solution containing SEQ ID No. 1 is then diluted to about 3/1 v/v water/ethanol to precipitate SEQ ID No. 1.

In an alternative embodiment, the aqueous ethanol solution containing SEQ ID NO. 1 is, instead of diluting with water, feed-stripped with ethanol in order to reduce the percentage of water in the solution until the percentage of ethanol is greater than 95%.

In another alternative embodiment, the aqueous ethanol solution containing SEQ ID NO. I is feed-stripped with ethanol in order to reduce the percentage of water in the solution until the percentage of ethanol is greater than 96%.

In another alternative embodiment, the aqueous ethanol solution containing SEQ ID NO. I is feed-stripped with ethanol in order to reduce the percentage of water in the solution until the percentage of ethanol is greater than 97%.

In another alternative embodiment, the aqueous ethanol solution containing SEQ ID NO. I is feed-stripped with ethanol in order to reduce the percentage of water in the solution until the percentage of ethanol is greater than 98%.

In another alternative embodiment, the ethanol solution containing SEQ ID NO. 1 is feed-stripped with heptane in order to reduce the percentage of ethanol in the solution until the percentage of heptane is greater than 98%.

In another alternative embodiment, the ethanol solution containing SEQ ID NO. 1 is feed-stripped with heptane in order to reduce the percentage of ethanol in the solution until the percentage of heptane is greater than 98.5%.

In another alternative embodiment, the ethanol solution containing SEQ ID NO. 1 is feed-stripped with heptane in order to reduce the percentage of ethanol in the solution until the percentage of heptane is greater than 99%.

In another alternative embodiment, the ethanol solution containing SEQ ID NO. 1 is feed-stripped with heptane in order to reduce the percentage of ethanol in the solution until the percentage of heptane is greater than 99.5%.

In another alternative embodiment, the ethanol solution containing SEQ ID NO. 1 is feed-stripped with heptane in order to reduce the percentage of ethanol in the solution until the percentage of heptane is greater than 99.9%.

In another alternative embodiment, the aqueous ethanol solution containing SEQ ID NO. 1, instead of diluting with water or feedstripping with ethanol and then heptane, is spray-dried.

The principles of the present invention will now be further illustrated with respect to the following examples. In the following all percentages and ratios are by volume unless otherwise expressly stated.

### EXAMPLES

### Example 1

Into a 1-neck, 3-L round bottom flask was placed 1500 mL ofa 50 % aqueous isopropanol solution of approximately 50.2 g SEQ ID NO. 1, which contained residual amounts of acetic acid and trifluoroacetic acid from HPLC chromatography. Analysis (HPLC, area normalization) of the initial product indicated a purity of 97.4 %.

The flask was placed under reduced pressure (34 Torr) on a rotary evaporator and partially immersed in a 30 °C bath. A granular, white suspension was formed by feed-stripping approximately 6500 mL of isopropanol over 396 minutes at such a rate as to maintain approximately 1500 mL of liquid in the 3-L flask. Analysis of the suspension (GC, area normalization) indicated water (1.8 %) and isopropanol (98.2 %).

The flask was placed under reduced pressure (46 Torr) on a rotary evaporator and partially immersed in a 30 °C bath. A more-granular white suspension was formed by feed-stripping approximately 6000 mL of heptane over 210 minutes at such a rate as to maintain approximately 1500 mL of liquid in the 3-L flask. Analysis (glc, area normalization) indicated water (0.0 %), isopropanol (0.0 %), and heptane (100.0 %).

Over two minutes, the granular suspension was vacuum filtered through a 600-mL, medium-glass frit (10-20 micron, 95 mm dia.) and the cake washed with 100 mL heptane to give a clear filtrate. Only 0.44 g of solids remained stuck to the 3-L flask.

Drying under reduced pressure (51 mTorr) at approximately 22 °C produced a granular solid containing isopropanol (0.8 wt. %) and heptane (2.5 wt. %).

The solid was placed in a glass-fritted resin reactor, and humidified by passing water-wet nitrogen through the solid. Analysis (GC) of the resulting granular solid indicated isopropanol (0.0 wt. %) and heptane (0.01 wt. %). Karl Fisher analysis indicated water (17.2 wt. %).

Drying the solid in the resin reactor under reduced pressure (29 Torr) with a nitrogen bleed at approximately 22 °C produced 39.2 g of white, granular SEQ ID NO. 1 containing water (2.7 wt. %).

Analysis (HPLC, area normalization) of the final, dried product indicated a purity of 97.0 %.

Table 1 below shows the amount of SEQ ID NO. 1 that is able to be dissolved in aqueous isopropanol solution, depending on the v/v% isopropanol:water. The very sharp increase in solubility between 40-60% isopropanol was very unexpected and the discovery of this unexpected result allowed for the successful isolation of SEQ ID NO. 1 by precipitation which was necessary for a viable large manufacturing scale-up required for commercial production.

**Table 1.**

| IPA (v/v %) | SEQ ID No. 1(mg/mL) |
|---|---|
| 0 | 1.3 |
| 5 | 1.8 |
| 10 | 2.3 |
| 15 | 3.3 |
| 20 | 4.3 |
| 25 | 4.8 |
| 30 | 6.0 |
| 35 | 7.0 |
| 40 | 13.8 |
| 45 | 47.8 |
| 50 | 120.3 |
| 55 | 162.5 |
| 60 | 138.5 |
| 65 | 106.5 |
| 70 | 65.7 |
| 75 | 26.5 |
| 80 | 19.0 |
| 85 | 12.5 |
| 90 | 9.0 |
| 95 | 4.5 |
| 100 | 0.3 |

### Example 2

The above general procedures were followed replacing isopropyl alcohol (IPA) with ethanol.

Table I below shows the amount of SEQ ID NO. 1 that is able to be dissolved in the aqueous ethanol solution, depending on the v/v% ethanol:water. The very sharp increase in solubility between 50-70 % ethanol was very unexpected and the discovery of this unexpected result allowed for the successful isolation of SEQ ID NO. 1 by precipitation which was necessary for a viable large manufacturing scale-up required for commercial production.

**Table 2.**

| EtOH (v/v %) | SEQ ID No. 1(mg/mL) |
|---|---|
| 0 | 0.50 |
| 5 | 0.25 |
| 10 | 0.75 |
| 15 | 1.00 |
| 20 | 0.75 |
| 25 | 3.00 |
| 30 | 3.00 |
| 35 | 2.75 |
| 40 | 2.25 |
| 45 | 3.50 |
| 50 | 9.25 |
| 55 | 179.25 |
| 60 | 347.50 |
| 65 | 430.00 |
| 70 | 441.50 |
| 75 | 304.00 |
| 80 | 45.25 |
| 85 | 10.25 |
| 90 | 10.00 |
| 95 | 3.75 |
| 100 | 0.50 |

### Example 3

### Precipitation and Isolation of SEQ ID No. I

The above-described procedures for isolating SEQ ID No. 1 involved removal of water by vacuum feed stripping with isopropanol or ethanol and then with heptane in a rotary evaporator. Solvent removal by rotary evaporation was utilized to minimize potential product degradation and yield loss due product adhering to the upper surface of the flask. This procedure furnished product which was very easy to handle and to filter. However, removal of residual solvents, particularly heptane, required "humidification" with a stream of water-wet nitrogen and subsequent treatment with dry nitrogen. The purity during this entire isolation procedure decreased from 97.4 % to 97.0 %.

The current process stream consists of SEQ ID No. 1 in approximately 50 % aqueous isopropanol. This ratio of water and isopropanol is used to ensure maximum product solubility. In order to minimize product degradation, this process stream is usually stored at approximately 2 °C, which causes some of the product to precipitate. To this process stream, is added water, which causes additional product to precipitate, and the product is isolated by filtration and drying. In order to achieve a maximum recovery level, enough water is added to achieve a final water/isopropanol ratio of 3/1. The preferred temperature range for the dilution with water over one hour was 3 °C to 5 °C. Stirring within this temperature range for approximately 30 minutes was followed by cooling to approximately -5 °C over approximately 30 minutes and stirring at this lower temperature for 5 h to 6 h prior to filtration through a cooled filter. After a small rinse of the precipitation vessel into the filter with aqueous isopropanol (3/1, v/v), only approximately 1 % of the product is lost as a film in the precipitation vessel. Aggregation was sufficient so that simple filtration produced a clear filtrate. Approximately 4 % of the product is dissolved in the filtrate. Analytical data indicates that very little degradation is occurring during this precipitation procedure.

### Example 4

### Spray-drying of Linaclotide:

The purified peptide solution obtained from the preparative HPLC step or from a concentration step is fed to the spray-dryer unit and is atomized in the drying chamber. The fine mist created by the atomizer mixes with the hot nitrogen stream (drying gas) and evaporation of the solvents from the droplets begins. The feed rate of the solution is adjusted to achieve the desired gas outlet temperature. The drying gas carries the fine powder through the drying chamber out to the cyclone. Because the residence time in the drying chamber is very short thermal degradation is minimized. The cyclone separates the powder from the drying gas and the powder is collected by gravity into drums.

The substantially powder-free gas flows into a filter bag housing where any very fine particles are retained in the bag filters. The powder free gas may be cooled down in a condenser where solvent condensation then occurs. The drying gas may either be re-circulated back to the drying chamber after re-heating or it may be used in a once through mode.

The composition of the feed solution (water versus organic solvents) determines the amount of energy that must be supplied to each droplet to evaporate essentially all the solvents. The droplet size in the spray-dryer along with the concentration of the peptide in the feed solution controls the size of the resulting particles and influences the recovery of the spray-drying process.

Exemplary spray-drying process parameters:

| | |
|---|---|
| Feed solution composition: | 1 -12 % (w/w) SEQ ID NO. 1 |
| | 10 - 80 % (w/w) water |
| | 90 - 20 % (w/w) ethanol or isopropanol |
| | 0 - 5 % (w/w) acetic acid |
| Atomizing mode: | Rotary wheel atomizer |
| Atomizer speed: | 5'000-50'000 RPM |
| Drying gas inlet temperature: | 100-160°C |
| Drying gas outlet temperature: | 50-90°C |
| Drying gas (nitrogen): | 300-500 kg/h, open or closed cycle modes. |

The features disclosed in the foregoing description, or the following claims, expressed in their specific forms or in terms of a means for performing the disclosed function, or a method or process for attaining the disclosed result, as appropriate, may, separately, or in any combination of such features, be utilized for realizing the invention in diverse forms thereof.

The foregoing invention has been described in some detail by way of illustration and example, for purposes of clarity and understanding. It will be obvious to one of skill in the art that changes and modifications may be practiced within the scope of the appended claims. Therefore, it is to be understood that the above description is intended to be illustrative and not restrictive. The scope of the invention should, therefore, be determined not with reference to the above description, but should instead be determined with reference to the following appended claims, along with the full scope of equivalents to which such claims are entitled.

## Claims

1. A process for precipitating a peptide comprising the step of:
a) providing an aqueous isopropanol or ethanol solution of a cyclized peptide including the amino acid sequence of (SEQ ID NO. 1)
Cys-Cys-Glu-Tyr-Cys-Cys-Asn-Pro-Ala-Cys-Thr-Gly-Cys-Tyr

2. The process of claim 1, wherein the alcohol solution is a isopropanol solution and the process further comprising the step of:
b) feed-stripping the aqueous solution resulting from step a) with isopropanol in order to reduce the percentage of water in the solution.

3. The process of claim 1, wherein the alcohol solution is a ethanol solution and the process further comprising the step of:
b) feed-stripping the solution resulting from step a) with ethanol in order to reduce the percentage of water in the solution.

4. The process of claim 2 or 3, wherein the feed-stripping with isopropanol or ethanol in step b) reduces the percentage of water in the solution until the percentage of isopropanol or ethanol is greater than 95%, preferably 96%, more preferably 97%, most preferably 98%.

5. The process of any one of claims 1 - 4, further comprising the step of:
c) feed-stripping the solution resulting from step b) with heptane in order to reduce the percentage of isopropanol or ethanol in the solution.

6. The process of claim 5, wherein the feed-stripping with heptane in step c) reduces the percentage of isopropanol or ethanol until the percentage of heptane is greater than 98%, preferably 98.5%, more preferably 99%, even more preferably 99.5%, most preferably 99.9%.

7. The process of claim 1, further comprising the step of:
b) diluting with water the solution resulting from step a).

8. The process of claim 7, wherein the solution is diluted to about 3/1 v/v water/isopropanol or to about 3/1 v/v water/ethanol to precipitate SEQ ID No. 1.

9. A process for isolating a peptide comprising the steps of:
a) providing an aqueous isopropanol or ethanol solution of a cyclized peptide including the amino acid sequence of (SEQ ID NO. 1)
Cys-Cys-Glu-Tyr-Cys-Cys-Asn-Pro-Ala-Cys-Thr-Gly-Cys-Tyr
b) spray drying the solution resulting from step a).

10. The process of any one of claims 1, 2, 4 - 6 and 9, wherein the aqueous isopropanol solution is an approximately 45% to approximately 70% aqueous isopropanol solution.

11. The process of claim 10, wherein the aqueous isopropanol solution is an approximately 45% isopropanol to approximately 65% aqueous isopropanol solution.

12. The process of claim 11, wherein the aqueous isopropanol solution is an approximately 45% isopropanol to approximately 60% aqueous isopropanol solution.

13. The process of claim 12, wherein the aqueous isopropanol solution is an approximately 50% to approximately 60% aqueous isopropanol solution.

14. The process of claim 13, wherein the aqueous isopropanol solution is an approximately 55% aqueous isopropanol solution.

15. The process of any one of claims 1, 3, 4 - 6 and 9, wherein the aqueous ethanol solution is an approximately 55% to approximately 75% aqueous ethanol solution.

16. The process of claim 15, wherein the aqueous ethanol solution is an approximately 60% to approximately 75% aqueous ethanol solution.

17. The process of claim 16, wherein the aqueous ethanol solution is an approximately 65% to approximately 75% aqueous ethanol solution.

## Patentansprüche

1. Verfahren zum Präzipitieren eines Peptids umfassend den Schritt:
a) Bereitstellen einer wässrigen Isopropanol- oder Ethanol-Lösung eines cyclisierten Peptids, das die Aminosäuresequenz der (SEQ ID NO. 1) einschließt
Cys-Cys-Glu-Tyr-Cys-Cys-Asn-Pro-Ala-Cys-Thr-Gly-Cys-Tyr.

2. Verfahren nach Anspruch 1, wobei die Alkohol-Lösung eine Isopropanol-Lösung ist und das Verfahren ferner den Schritt umfasst:
b) Zufuhr-Entfernung der in Schritt a) erhaltenen wässrigen Lösung mit Isopropanol, um den Anteil von Wasser in der Lösung zu reduzieren.

3. Verfahren nach Anspruch 1, wobei die Alkohol-Lösung eine Ethanol-Lösung ist und das Verfahren ferner den Schritt umfasst:
b) Zufuhr-Entfernung der aus Schritt a) erhaltenen wässrigen Lösung mit Ethanol, um den Anteil von Wasser in der Lösung zu reduzieren.

4. Verfahren nach Anspruch 2 oder 3, wobei die Zufuhr-Entfernung mit Isopropanol oder Ethanol in Schritt b) den Anteil von Wasser in der Lösung reduziert, bis der Anteil von Isopropanol oder Ethanol größer als 95%, vorzugsweise 96%, mehr bevorzugt 97%, am meisten bevorzugt 98% ist.

5. Verfahren nach einem der Ansprüche 1-4, ferner umfassend den Schritt:
c) Zufuhr-Entfernung der in Schritt b) erhaltenen wässrigen Lösung mit Heptan, um den Anteil von Isopropanol oder Ethanol in der Lösung zu reduzieren.

6. Verfahren nach Anspruch 5, wobei die Zufuhr-Entfernung mit Heptan in Schritt c) den Anteil von Isopropanol oder Ethanol reduziert, bis der Anteil von Heptan größer als 98%, vorzugsweise 98.5%, mehr bevorzugt 99%, noch mehr bevorzugt 99.5%, am meisten bevorzugt 99.9% ist.

7. Verfahren nach Anspruch 1, ferner umfassend den Schritt:
b) Verdünnen der in Schritt a) erhaltenen Lösung mit Wasser.

8. Verfahren nach Anspruch 7, wobei die Lösung auf etwa 3/1 Vol./Vol. Wasser/Isopropanol oder auf etwa 3/1 Vol./Vol. Wasser/Ethanol verdünnt ist, um die SEQ ID NO. 1 zu präzipitieren.

9. Verfahren zur Isolierung eines Peptids, umfassend die Schritte:
a) Bereitstellen einer wässrigen Isopropanol- oder Ethanol-Lösung eines cyclisierten Peptids, das die Aminosäuresequenz der (SEQ ID NO. 1) einschließt
Cys-Cys-Glu-Tyr-Cys-Cys-Asn-Pro-Ala-Cys-Thr-Gly-Cys-Tyr
b) Sprühtrocknen der in Schritt a) erhaltenen Lösung.

10. Verfahren nach einem der Ansprüche 1, 2, 4-6 und 9, wobei die wässrige Isopropanol-Lösung eine etwa 45%ige bis etwa 70%ige wässrige Isopropanol-Lösung ist.

11. Verfahren nach Anspruch 10, wobei die wässrige Isopropanol-Lösung eine etwa 45%ige Isopropanol- bis etwa 65%ige wässrige Isopropanol-Lösung ist.

12. Verfahren nach Anspruch 11, wobei die wässrige Isopropanol-Lösung eine etwa 45%ige Isopropanol- bis etwa 60%ige wässrige Isopropanol-Lösung ist.

13. Verfahren nach Anspruch 12, wobei die wässrige Isopropanol-Lösung eine etwa 50%ige bis etwa 60%ige wässrige Isopropanol-Lösung ist.

14. Verfahren nach Anspruch 13, wobei die wässrige Isopropanol-Lösung eine etwa 55%ige wässrige Isopropanol-Lösung ist.

15. Verfahren nach einem der Ansprüche 1, 3, 4-6 und 9, wobei die wässrige Ethanol-Lösung eine etwa 55%ige bis etwa 75%ige wässrige Ethanol-Lösung ist.

16. Verfahren nach Anspruch 15, wobei die wässrige Ethanol-Lösung eine etwa 60%ige bis etwa 75%ige wässrige Ethanol-Lösung ist.

17. Verfahren nach Anspruch 16, wobei die wässrige Ethanol-Lösung eine etwa 65%ige bis etwa 75%ige wässrige Ethanol-Lösung ist.

## Revendications

1. Procédé pour précipiter un peptide, comprenant l'étape consistant à :
a) mettre à disposition une solution aqueuse d'isopropanol ou d'éthanol d'un peptide cyclisé comprenant la séquence d'acides aminés de (SEQ ID NO: 1)
Cys-Cys-Glu-Tyr-Cys-Cys-Asn-Pro-Ala-Cys-Thr-Gly-Cys-Tyr

2. Procédé selon la revendication 1, dans lequel la solution d'alcool est une solution d'isopropanol et le procédé comprend en outre l'étape consistant à :
b) rectifier la solution aqueuse résultant de l'étape a) avec de l'isopropanol afin de réduire le pourcentage d'eau dans la solution.

3. Procédé selon la revendication 1, dans lequel la solution d'alcool est une solution d'éthanol et le procédé comprend en outre l'étape consistant à :
b) rectifier la solution résultant de l'étape a) avec de l'éthanol afin de réduire le pourcentage d'eau dans la solution.

4. Procédé selon la revendication 2 ou 3, dans lequel la rectification avec de l'isopropanol ou de l'éthanol à l'étape b) réduit le pourcentage d'eau dans la solution jusqu'à ce que le pourcentage d'isopropanol ou d'éthanol soit supérieur à 95 %, de préférence 96 %, de manière davantage préférée 97 %, de la manière la plus préférée entre toutes 98 %.

5. Procédé selon l'une quelconque des revendications 1 à 4, comprenant en outre l'étape consistant à :
c) rectifier la solution résultant de l'étape b) avec de l'heptane afin de réduire le pourcentage d'isopropanol ou d'éthanol dans la solution.

6. Procédé selon la revendication 5, dans lequel la rectification avec de l'heptane à l'étape c) réduit le pourcentage d'isopropanol ou d'éthanol jusqu'à ce que le pourcentage d'heptane soit supérieur à 98 %, de préférence 98,5 %, de manière davantage préférée 99 %, encore de manière davantage préférée 99,5 %, de la manière la plus préférée entre toutes 99,9 %.

7. Procédé selon la revendication 1, comprenant en outre l'étape consistant à :
b) diluer la solution résultant de l'étape a) avec de l'eau.

8. Procédé selon la revendication 7, dans lequel la solution est diluée à un rapport eau/isopropanol d'environ 3/1 v/v ou à un rapport eau/éthanol d'environ 3/1 v/v afin de précipiter SEQ ID NO: 1.

9. Procédé pour isoler un peptide, comprenant les étapes consistant à :
a) mettre à disposition une solution aqueuse d'isopropanol ou d'éthanol d'un peptide cyclisé comprenant la séquence d'acides aminés de (SEQ ID NO: 1)
Cys-Cys-Glu-Tyr-Cys-Cys-Asn-Pro-Ala-Cys-Thr-Gly-Cys-Tyr
b) sécher par pulvérisation la solution résultant de l'étape a).

10. Procédé selon l'une quelconque des revendications 1, 2, 4 à 6 et 9, dans lequel la solution aqueuse d'isopropanol est une solution aqueuse d'isopropanol d'approximativement 45 % à approximativement 70 %.

11. Procédé selon la revendication 10, dans lequel la solution aqueuse d'isopropanol est une solution aqueuse d'isopropanol d'approximativement 45 % à approximativement 65 %.

12. Procédé selon la revendication 11, dans lequel la solution aqueuse d'isopropanol est une solution aqueuse d'isopropanol d'approximativement 45 % à approximativement 60 %.

13. Procédé selon la revendication 12, dans lequel la solution aqueuse d'isopropanol est une solution aqueuse d'isopropanol d'approximativement 50 % à approximativement 60 %.

14. Procédé selon la revendication 13, dans lequel la solution aqueuse d'isopropanol est une solution aqueuse d'isopropanol d'approximativement 55 %.

15. Procédé selon l'une quelconque des revendications 1, 3, 4 à 6 et 9, dans lequel la solution aqueuse d'éthanol est une solution aqueuse d'éthanol d'approximativement 55 % à approximativement 75 %.

16. Procédé selon la revendication 15, dans lequel la solution aqueuse d'éthanol est une solution aqueuse d'éthanol d'approximativement 60 % à approximativement 75 %.

17. Procédé selon la revendication 16, dans lequel la solution aqueuse d'éthanol est une solution aqueuse d'éthanol d'approximativement 65 % à approximativement 75 %.


### SEQUENCE LISTING

<110> F. Hoffmann-La Roche AG
<120> Process for isolating therapeutic peptide
<130> 26071 WO
<150> US 61/168259
   <151> 2009-04-10
<160> 1
<170> PatentIn version 3.5
<210> 1
   <211> 14
   <212> PRT
   <213> Artificial Sequence
<220>
   <223> Linaclotide
<400> 1
   Cys Cys Glu Tyr Cys Cys Asn Pro Ala Cys Thr Gly Cys Tyr
   1               5                   10